# EUROPÄISCHE PATENTSCHRIFT

(11) **EP 4 559 403 B1**
(45) Veröffentlichungstag und Bekanntmachung des Hinweises auf die Patenterteilung: **05.08.2026**
(21) Anmeldenummer: 24212695.1
(22) Anmeldetag: 13.11.2024
(51) Int. Cl.: A61B 6/03, A61B 6/00

(54) **VERFAHREN ZUM VORBEREITEN EINER REINIGUNGSEINHEIT MITTELS EINER VORBEREITUNGSSTATION**
METHOD FOR PREPARING A CLEANING UNIT BY MEANS OF A PREPARATION STATION
PROCÉDÉ DE PRÉPARATION D'UNE UNITÉ DE NETTOYAGE AU MOYEN D'UNE STATION DE PRÉPARATION

(30) Priorität: 24.11.2023 AT 509502023
(43) Veröffentlichungstag der Anmeldung: 28.05.2025
(73) Patentinhaber: Hagleitner, Hans Georg, 5700 Zell am See (AT)
(72) Erfinder: Hagleitner, Hans Georg, 5700 Zell am See (AT)
(74) Vertreter: Torggler & Hofmann Patentanwälte - Innsbruck

(56) Entgegenhaltungen:
- DE-A1- 102021 210 471
- US-A1- 2022 143 240

## Beschreibung

Die vorliegende Erfindung betrifft
- Verfahren zum Vorbereiten einer Reinigungseinheit und zum Reinigen einer einen Patientenaufnahmebereich umgebenden Umhausung einer medizinischen Bildgebungsvorrichtung mittels einer solchen Reinigungseinheit und mittels einer Vorbereitungsstation für ein Verfahren zum Vorbereiten und/oder Reinigen einer solchen Reinigungseinheit
- eine solche Vorbereitungsstation und
- eine Anordnung mit einer solchen Vorbereitungsstation.

Medizinischen Bildgebungsvorrichtungen werden in der Radiologie verwendet und sind zum Beispiel MRT- und CT-Geräte. Diese werden bei Verwendung verschmutzt und/oder mit Keimen und/oder Viren kontaminiert. Dies kann beispielsweise durch die zu untersuchenden Personen selbst innerhalb der medizinischen Bildgebungsvorrichtung erfolgen, wenn bei Husten, Niesen, feuchter Atmung, physischer Berührung der medizinischen Bildgebungsvorrichtung oder Ähnlichem Keime oder andere Verschmutzungen hinterlassen werden. Derartige Verschmutzungen verbleiben unter anderem auf der Innenseite, konkret einer Umhausung, der medizinischen Bildgebungsvorrichtung.

Eine manuelle Reinigung erfolgt hierbei mit einem optional an einer Stange befestigen Reinigungsutensil, wie beispielsweise in US 5918342 offenbart.

Nachteilig dabei ist, dass diese Art der Reinigung sehr umständlich und zeitaufwändig ist und/oder sich das Reinigungspersonal sehr nahe oder sogar teilweise innerhalb der medizinischen Bildgebungsvorrichtung aufhält. Der radiologische Einfluss, wie zum Beispiel Röntgenstrahlung oder Magnetfelder, der medizinischen Bildgebungsvorrichtung kann dabei unangenehm sein.

Es ist weiters bekannt, automatische Reinigungseinheiten innerhalb der medizinischen Bildgebungsvorrichtung vorzusehen, wie beispielsweise in CN 219251060 U oder DE 102021210471 A1 offenbart. Dabei kann die Reinigungseinheit eine Wischreinigung durchführen, indem die Reinigungseinheit von einem Ende der Umhausung zum anderen Ende der Umhausung verfahren wird.

Nachteilig dabei ist, dass das Vorbereiten einer derartigen Reinigungseinheit zeitintensiv und aufwändig ist. Während der Vorbereitung kann die medizinische Bildgebungsvorrichtung nicht oder nur begrenzt verwendet werden, wodurch die Betriebszeit der medizinischen Bildgebungsvorrichtung einschränkt wird.

Die Aufgabe der vorliegenden Erfindung besteht also darin, die Nachteile des Standes der Technik zumindest teilweise zu beheben und ein gegenüber dem Stand der Technik verbessertes Verfahren zum Vorbereiten einer Reinigungseinheit anzugeben, wobei sich das Verfahren insbesondere durch einen geringeren Aufwand für das Vorbereitungspersonal auszeichnet. Die Aufgabe besteht weiterhin darin, ein Verfahren zum Reinigen einer Reinigungseinheit, eine Vorbereitungsstation für ein Verfahren zum Vorbereiten und/oder Reinigen einer solchen Reinigungseinheit und eine Anordnung mit einer solchen Vorbereitungsstation anzugeben.

Diese Aufgabe wird gelöst durch die Merkmale der Ansprüche 1, 5 und 14.

Diese Aufgabe wird mittels einem Verfahren nach Anspruch 1 gelöst, nämlich ein Verfahren zum Vorbereiten einer Reinigungseinheit und zum Reinigen einer einen Patientenaufnahmebereich umgebenden Umhausung einer medizinischen Bildgebungsvorrichtung
- mittels einer Reinigungseinheit, wobei die Reinigungseinheit eine Trägerstruktur und eine Reinigungskomponente aufweist, und
- mittels einer, bevorzugt von der medizinischen Bildgebungsvorrichtung getrennten und in der Nachbarschaft derselben angeordneten, Vorbereitungsstation mit zumindest einer Vorbereitungsvorrichtung und mit zumindest einer Verfahrvorrichtung, wobei die Reinigungseinheit mit der Vorbereitungsstation lösbar verbunden ist,
   o wobei zumindest ein Vorbereitungsschritt durch die zumindest eine Vorbereitungsvorrichtung durchgeführt wird, durch welchen die Reinigungseinheit für eine nachfolgende Reinigung vorbereitet wird und
   o wobei die Reinigung durch ein Bewegen der vorbereiteten Reinigungseinheit innerhalb der medizinischen Bildgebungsvorrichtung in zumindest eine Richtung, bevorzugt in zumindest zwei Richtungen, erfolgt.

Für das Vorbereiten einer Reinigungseinheit sind oftmals verschiedene Vorbereitungsschritte notwendig. Wenn eine Reinigungseinheit direkt an der medizinischen Bildgebungsvorrichtung für eine neue Bildgebung vorbereitet wird, so erhöht sich zumindest der Zeitaufwand, bis die neue Bildgebung durchgeführt werden kann. Beispielsweise muss ein Patient warten, bis die Reinigungseinheit fertig vorbereitet ist, bevor dieser auf einen Patiententisch Platz nehmen kann. Durch das erfindungsgemäße Verfahren kann mittels einer von der medizinischen Bildgebungsvorrichtung getrennten und in der Nachbarschaft derselben angeordneten Vorbereitungsstation die Reinigungseinheit so vorbereitet werden, dass währenddessen die medizinische Bildgebungsvorrichtung zumindest teilweise zugänglich und zumindest teilweise verwendbar ist. Beispielsweise kann das Verfahren zum Vorbereiten der Reinigungseinheit während eines Patientenwechsels stattfinden. Insbesondere bei älteren und gebrechlichen Patienten erfordert ein Patientenwechsel Zeit, in der gleichzeitig das Vorbereiten der Reinigungseinheit erfolgen kann und somit ein geringerer Zeitaufwand erreicht wird, weil die Reinigungseinheit an der Vorbereitungsstation vorbereitet wird und der Patientenaufnahmebereich leichter zugänglich ist.

Ein Vorteil eines solchen Verfahrens zum Vorbereiten der Reinigungseinheit ist, dass zumindest ein Vorbereitungsschritt für das Vorbereiten der Reinigungseinheit mittels der Vorbereitungsstation erfolgen kann. Die Vorbereitungsstation erleichtert somit zumindest einen, bevorzugt vollständig manuellen, Vorbereitungsschritt und verringert somit den Vorbereitungsaufwand für das Vorbereitungspersonal.

Unter "Reinigen" wird hier Säubern, Desinfizieren und/oder Sterilisieren verstanden. Das Reinigen kann dabei in Form von Wischen, Abschaben, Abkratzen, Reiben, Schrubben, Waschen und/oder dergleichen erfolgen.

Unter "in der Nachbarschaft derselben angeordneten Vorbereitungsstation" wird hier verstanden, dass sich die Vorbereitungsstation in der Nähe der medizinischen Bildgebungsvorrichtung befindet, wobei der Abstand zwischen der Vorbereitungsstation und der medizinischen Bildgebungsvorrichtung ausreichend dafür ist, dass der radiologische Einfluss, zum Beispiel von Röntgenstrahlung oder von einem Magnetfeld der medizinischen Bildgebungsvorrichtung, nicht negativ auf die Vorbereitungsstation, insbesondere metallische Bauteile und/oder elektronische Bauteile der Vorbereitungsstation, und/oder auf das Vorbereitungspersonal beim Durchführen eines Verfahrens zur Vorbereitung der Reinigungseinheit auswirkt. Ein ausreichender Abstand zwischen der Vorbereitungsstation und der medizinischen Bildgebungsvorrichtung kann beispielsweise mindestens 0,5 Meter sein.

Unter "Vorbereitungsschritte" werden hier alle Maßnahmen verstanden, welche an einer Reinigungseinheit vor, während und/oder nach einer medizinischen Bildgebung durchgeführt werden.

Es kann vorgesehen sein, dass die Reinigungseinheit vor und/oder nach dem zumindest einen Vorbereitungsschritt in der Vorbereitungsstation geparkt wird.

Der Vollständigkeit halber wird darauf hingewiesen, dass im Zuge der Beschreibung dieser Erfindung die verwendeten Zahlwörter wie ein, zwei, drei und dergleichen, grundsätzlich nur die vorhandene Mindestmenge eines Merkmals der erfindungsgemäßen Verfahren, der erfindungsgemäßen Vorbereitungsstation und/oder der erfindungsgemäßen Anordnung mit einer solchen Vorbereitungsstation beschreibt. Einzelne Merkmale oder Bestandteile können natürlich auch in größerer Anzahl vorhanden sein. So können beispielsweise erfindungsgemäße Verfahren mehr als einen Vorbereitungsschritt und eine erfindungsgemäße Vorbereitungsstation mehr als eine Vorbereitungsvorrichtung usw. aufweisen. In diesem Sinne ist also beispielsweise das Zahlwort ein soweit sinnvoll im Sinne von mindestens ein usw. zu verstehen.

In einer bevorzugten Ausführungsvariante kann vorgesehen sein, dass das Verfahren zum Vorbereiten einer Reinigungseinheit und/oder das Verfahren zum Reinigen einer einen Patientenaufnahmebereich umgebenden Umhausung einer medizinischen Bildgebungsvorrichtung zumindest einen oder zumindest zwei oder zumindest drei oder zumindest vier oder zumindest fünf Vorbereitungsschritte umfasst.

Beispielsweise können zumindest drei Vorbereitungsschritte vorgesehen sein, wobei das Verformen der Reinigungskomponente, das Platzieren der Reinigungsauflage und das Aufbringen und/oder Einbringen eines Reinigungsmittels jeweils ein Vorbereitungsschritt sein kann.

In einem anderen Beispiel kann zusätzlich zum vorigen Beispiel ein vierter Vorbereitungsschritt vorgesehen sein, wobei ein zweites Mal das Reinigungsmittel oder ein anderes Reinigungsmittel zusätzlich aufgebracht und/oder eingebracht wird.

Die einzelnen hier offenbarten Vorbereitungsschritte können im Zuge des Verfahrens zum Vorbereiten einer Reinigungseinheit und/oder des Verfahrens zum Reinigen einer einen Patientenaufnahmebereich umgebenden Umhausung einer medizinischen Bildgebungsvorrichtung wiederholt und/oder miteinander kombiniert werden.

In einer bevorzugten Ausführungsvariante kann vorgesehen sein, dass die Vorbereitungsstation zumindest eine oder zumindest zwei oder zumindest drei oder zumindest vier oder zumindest fünf Vorbereitungsvorrichtungen umfasst.

Beispielsweise können zumindest drei Vorbereitungsvorrichtungen vorgesehen sein, wobei das die Verformungsvorrichtung, die Platziervorrichtung und die Aufbringvorrichtung jeweils eine Vorbereitungsvorrichtung sein kann.

In einem anderen Beispiel kann zusätzlich zum vorigen Beispiel eine vierte Vorbereitungsvorrichtung vorgesehen sein, wobei zusätzlich eine zweite Aufbringvorrichtung für ein zweites Aufbringen und/oder Einbringen des Reinigungsmittels oder für ein Aufbringen eines anderen Reinigungsmittels vorgesehen sein kann.

Die einzelnen hier offenbarten Vorbereitungsvorrichtungen können in der Vorbereitungsstation zum Vorbereiten einer Reinigungseinheit und/oder zum Reinigen einer einen Patientenaufnahmebereich umgebenden Umhausung einer medizinischen Bildgebungsvorrichtung mehrmals vorgesehen sein und/oder miteinander kombiniert werden.

In einer bevorzugten Ausführungsvariante kann vorgesehen sein, dass das Verfahren zumindest teilweise, bevorzugt vollständig, automatisiert ist.

Weitere vorteilhafte Ausführungsformen des Verfahrens werden in den abhängigen Ansprüchen definiert.

Gemäß einer bevorzugten Ausführungsform des Verfahrens ist es vorgesehen, dass
- die Reinigungseinheit zu der Vorbereitungsstation hinbewegt wird und
- anschließend zumindest ein Vorbereitungsschritt durchgeführt wird, durch welchen die Reinigungseinheit für eine nachfolgende Reinigung vorbereitet wird.

Gemäß einer bevorzugten Ausführungsform des Verfahrens ist es vorgesehen, dass zumindest einer der folgenden Verfahrensschritte vorgesehen ist:
- lösbares Verbinden der Reinigungseinheit mit der Vorbereitungsstation, bevorzugt mittels einer Kopplungsvorrichtung,
- Aufladen eines Kraftspeichers und/oder eines Energiespeichers,
- Verformen, bevorzugt Aufblasen, der Reinigungskomponente durch Befüllen mit einem Druckmedium oder durch Entnahme des Druckmediums,
- Platzieren, bevorzugt Aufspannen, einer Reinigungsauflage der Reinigungskomponente auf einen Reinigungsgrundkörper der Reinigungskomponente, bevorzugt entlang einer äußeren Kontur des Reinigungsgrundkörpers,
- Aufbringen und/oder Einbringen eines Reinigungsmittels auf und/oder in eine Reinigungsauflage der Reinigungskomponente, bevorzugt vor, während und/oder nach einem Platzieren der Reinigungsauflage der Reinigungskomponente auf einen Reinigungsgrundkörper der Reinigungskomponente.

Vorbereitungsschritte wie zum Beispiel ein Verformen der Reinigungskomponente, ein Platzieren der Reinigungsauflage und/oder ein Aufbringen und/oder Einbringen eines Reinigungsmittels können zeitaufwändig und/oder arbeitsaufwändig sein, weshalb der Einsatz einer Vorbereitungsstation im Zuge eines Verfahrens zum Vorbereiten einer Reinigungseinheit die Totzeit, konkret die ungenützte Zeit, verringert und/oder die Betriebszeit einer medizinischen Bildgebungsvorrichtung besser und effizienter nützt.

Ein lösbares Verbinden der Reinigungseinheit mit der Vorbereitungsstation kann im einfachsten Fall vorliegen, wenn die Reinigungseinheit auf oder in die Vorbereitungsstation gestellt wird. Es kann stattdessen oder zusätzlich vorgesehen sein, dass eine eigene Kopplungsvorrichtung zum lösbaren Verbinden vorgesehen ist.

Unter "lösbares Verbinden", "lösbar verbunden" und "lösbar verbindbar" kann verstanden werden, dass zumindest zwei ursprünglich voneinander getrennte Komponenten miteinander verbunden werden können, wobei dieses lösbare Verbinden anschließend zerstörungsfrei aufgelöst werden kann, sodass die zumindest zwei Komponenten wieder in ihrem ursprünglichen voneinander getrennten Zustand vorliegen, um daraufhin erneut verbunden werden zu können. "Lösbares Verbinden", "lösbar verbunden" und "lösbar verbindbar" kann daher auch als "zerstörungsfrei lösbares Verbinden", "zerstörungsfrei lösbar verbunden" und "zerstörungsfrei lösbar verbindbar" bezeichnet werden.

Ein Energiespeicher dient der Speicherung von momentan verfügbarer, aber nicht benötigter Energie zur späteren Nutzung. Ein Energiespeicher kann ein elektrischer Energiespeicher wie zum Beispiel eine Batterie sein. Ein Kraftspeicher dient analog zum Bereitstellen einer Kraft zur späteren Nutzung. Ein Kraftspeicher kann ein mechanischer Kraftspeicher wie zum Beispiel eine Federvorrichtung sein.

Mit Hilfe eines Energiespeichers oder eines Kraftspeichers kann zumindest ein Vorbereitungsschritt zum Vorbereiten der Reinigungseinheit und/oder ein Reinigungsschritt zum Reinigen einer einen Patientenaufnahmebereich umgebenden Umhausung zu einem späteren Zeitpunkt ausgeführt werden. Beispielsweise kann eine Feder als Kraftspeicher beim Vorbereiten aufgeladen werden, um später, bevorzugt wenn die Reinigungseinheit während des Reinigens innerhalb der Umhausung angeordnet ist, ein Verformen der Reinigungskomponente hervorzurufen. Auf diese Weise kann die Reinigungskomponente der Reinigungseinheit durch die aufgeladene Feder innerhalb der Umhausung gegen die Umhausung gepresst oder aber das Anpressen beendet werden.

Unter "Verformen" und/oder "verformbar" wird hier verstanden, dass die Form eines Körper infolge der Einwirkung einer Kraft und/oder einer Spannung verändert werden kann. Eine Verformung eines Körper kann in einen ersten Anteil, bei dem sich das Volumen und/oder die räumliche Ausdehnung des Körpers aber nicht die geometrische Form des Körpers ändert, beispielsweise bei einer Radiusvergrößerung einer Kugel oder eines schlauchförmigen Körpers, und in einen zweiten Anteil, bei dem sich die geometrische Form des Körpers aber nicht das Volumen und/oder die räumliche Ausdehnung des Körpers ändert, beispielsweise bei einer Dehnung einer Kugel entlang einer Achse zu einem Ellipsoid, zerlegt werden. Bei einer Verformung durch Befüllung der Reinigungskomponente mit einem Druckmedium kann eine Verformung mit einem ersten und/oder einem zweiten Anteil erfolgen.

Die Verformbarkeit der Reinigungskomponente durch Befüllen mit einem Druckmedium bewirkt, dass eine geometrische Form, bevorzugt ein Außendurchmesser, die räumliche Ausdehnung und/oder das Volumen der Reinigungskomponente geändert wird. Auf diese Weise kann ein Kontaktieren der in der medizinischen Bildgebungsvorrichtung angeordneten Reinigungseinheit mit der Umhausung der medizinischen Bildgebungsvorrichtung gezielt hervorgerufen oder unterlassen werden.

Somit kann eine zuverlässige Reinigung einer Umhausung durchgeführt werden, indem die Reinigungskomponente in einen befüllten Zustand versetzt wird, die Umhausung durch die befüllte Reinigungskomponente kontaktiert wird und die Reinigungskomponente beim Bewegen der Reinigungseinheit die Umhausung beispielsweise durch Wischen reinigt.

Eine "nicht-befüllte Reinigungskomponente" oder eine "Reinigungskomponente in einem nicht-befüllten Zustand" ist eine Reinigungskomponente, welche nicht mit einem Druckmedium befüllt ist, wobei die nicht-befüllte Reinigungskomponente einer in die Umhausung eingeführten Reinigungseinheit die Umhausung nicht kontaktiert.

In einem nicht-befüllten Zustand steht die Reinigungskomponente unter der Kraft- und/oder Spannungseinwirkung des die Reinigungskomponente umgebenden Atmosphärendrucks und optional eines in der Reinigungskomponente vorhandenen Mediums, welches nicht das Druckmedium ist, aber keinesfalls unter der Druckwirkung der Umhausung.

Eine "teilweise befüllte Reinigungskomponente" oder eine "Reinigungskomponente in einem teilweise befüllten Zustand" ist eine Reinigungskomponente, welche zumindest teilweise mit einem Druckmedium befüllt ist, wobei die teilweise befüllte Reinigungskomponente einer in die Umhausung eingeführten Reinigungseinheit die Umhausung nicht kontaktiert.

Eine "befüllte Reinigungskomponente" oder eine "Reinigungskomponente in einem befüllten Zustand" ist eine Reinigungskomponente, welche mit einem Druckmedium befüllt ist, wobei die befüllte Reinigungskomponente einer in die Umhausung eingeführten Reinigungseinheit die Umhausung kontaktiert.

In einem befüllten Zustand steht die Reinigungskomponente unter der Kraft- und/oder Spannungseinwirkung des Druckmediums, des die Reinigungskomponente umgebenden Atmosphärendrucks und der die Reinigungskomponente kontaktierenden Umhausung und optional einer Reaktionskraft der, bevorzugt elastischen, Reinigungskomponente infolge der Verformung.

Beim Übergang einer befüllten Reinigungskomponente, bei der ein Kontaktieren der Umhausung vorliegt, über eine teilweise befüllte Reinigungskomponente bis hin zu einer nicht-befüllten Reinigungskomponente wird Druckmedium aus der Reinigungskomponente entnommen, wodurch sich eine räumliche Ausdehnung der Reinigungskomponente verringert und das Kontaktieren der Umhausung beendet wird.

Beim Übergang einer nicht-befüllten Reinigungskomponente, bei der kein Kontaktieren der Umhausung vorliegt, über eine teilweise befüllte Reinigungskomponente bis hin zu einer befüllten Reinigungskomponente wird Druckmedium in die Reinigungskomponente eingeführt, wodurch sich eine räumliche Ausdehnung der Reinigungskomponente vergrößert und das Kontaktieren der Umhausung erreicht wird.

Erst wenn ausreichend Druckmedium in eine Reinigungskomponente einer Reinigungseinheit eingeführt ist oder, mit anderen Worten, wenn die Reinigungskomponente ausreichend mit Druckmedium befüllt ist, findet der Übergang von der teilweise befüllten Reinigungskomponente hin zur befüllten Reinigungskomponente statt, wodurch ein Kontaktieren der Umhausung durch eine in die Umhausung eingeführten Reinigungseinheit ermöglicht wird. Wird anschließend eine bereits befüllte Reinigungskomponente noch weiter mit Druckmedium befüllt, so liegt ein Kontaktieren der Umhausung in Form eines Anpressens vor, wobei der Anpressdruck durch den Grad der Befüllung der befüllten Reinigungskomponente bestimmt wird.

Eine Reinigungskomponente kann mehrmals hintereinander befüllt werden und bei jeder neuen Befüllung seine räumliche Ausdehnung entsprechend obigen Ausführungen vergrößern. Dies gilt analog für eine Reinigungskomponente, der mehrmals hintereinander ein Teil des Druckmediums entnommen wird.

Unter "Reinigungsmittel" kann hier ein allgemein bekanntes Mittel zum Säubern, beispielsweise Seife, ein Desinfektionsmittel, beispielsweise Alkohol, und/oder ein Sterilisationsmittel verstanden werden.

Gemäß einer bevorzugten Ausführungsform des Verfahrens ist es vorgesehen, dass zumindest einer der folgenden Verfahrensschritte vorgesehen ist:
- Lösen der mit der Vorbereitungsstation lösbar verbundenen Reinigungseinheit, bevorzugt mittels der Kopplungsvorrichtung,
- Transferieren der Reinigungseinheit zwischen der medizinischen Bildgebungsvorrichtung und der Vorbereitungsstation,
- Lösbares Verbinden der Reinigungseinheit mit der medizinischen Bildgebungsvorrichtung,
- Lösen der Reinigungseinheit von der medizinischen Bildgebungsvorrichtung.

In einer bevorzugten Ausführungsvariante kann vorgesehen sein, dass das Transferieren der Reinigungseinheit mittels einer Transfervorrichtung erfolgt.

In einer bevorzugten Ausführungsvariante des Verfahrens zum Reinigen einer einen Patientenaufnahmebereich umgebenden Umhausung einer medizinischen Bildgebungsvorrichtung kann vorgesehen sein, dass die zumindest eine Verfahrvorrichtung Teil der Vorbereitungsstation oder dass die zumindest eine Verfahrvorrichtung Teil der medizinischen Bildgebungsvorrichtung, beispielsweise ein Patiententisch, ist.

Mittels einer Vorbereitungsstation kann ein weniger aufwändiger und effizienterer Arbeitsablauf erreicht werden, indem die Vorbereitungsstation einerseits die Vorbereitung einer Reinigungseinheit erleichtert und andererseits als Verfahrvorrichtung zum Reinigen der Umhausung genützt werden kann.

In einer bevorzugten Ausführungsvariante kann, sobald das Bewegen der vorbereiteten Reinigungseinheit entlang der Umhausung in eine erste Richtung beendet ist, die befüllte Reinigungskomponente in einen nicht-befüllten Zustand versetzt werden und die Reinigungseinheit mit der nicht-befüllten Reinigungskomponente kontaktlos wieder zurück in die Ausgangsposition bewegt werden. Es kommt dabei zu keinem erneuten Kontakt der Reinigungskomponente mit der Umhausung, womit eine Rekontamination vermieden wird.

Weiters wird Schutz begehrt für eine Vorbereitungsstation zum Vorbereiten einer Reinigungseinheit, wobei die Reinigungseinheit zum Reinigen einer einen Patientenaufnahmebereich umgebenden Umhausung einer medizinischen Bildgebungsvorrichtung vorgesehen ist, wobei die Reinigungseinheit eine Trägerstruktur und eine Reinigungskomponente aufweist, wobei die Vorbereitungsstation von der medizinischen Bildgebungsvorrichtung getrennt und in der Nachbarschaft derselben angeordnet ist, wobei die Vorbereitungsstation zumindest eine Vorbereitungsvorrichtung aufweist, durch welche die Reinigungseinheit für eine nachfolgende Reinigung vorbereitet wird.

Weitere vorteilhafte Ausführungsformen der Vorbereitungsstation werden in den abhängigen Ansprüchen definiert.

Gemäß einer bevorzugten Ausführungsform des Vorbereitungsstation ist es vorgesehen, dass die zumindest eine Vorbereitungsvorrichtung
- eine Kopplungsvorrichtung zum lösbaren Verbinden der Reinigungseinheit mit der Vorbereitungsstation,
- eine Ladevorrichtung zum Aufladen eines Kraftspeichers und/oder eines Energiespeichers,
- eine Verformungsvorrichtung zum Verformen der Reinigungskomponente,
- eine Platziervorrichtung zum Platzieren einer Reinigungsauflage der Reinigungskomponente auf einen Reinigungsgrundkörper der Reinigungskomponente oder
- eine Aufbringvorrichtung zum Aufbringen eines Reinigungsmittels auf und/oder in eine Reinigungsauflage der Reinigungskomponente ist.

In einer bevorzugten Ausführungsvariante kann vorgesehen sein, dass die Vorbereitungsstation mit der Reinigungseinheit lösbar verbunden oder verbindbar ist.

In einer bevorzugten Ausführungsvariante kann vorgesehen sein, dass zumindest eine der Vorbereitungsvorrichtungen zumindest teilweise, bevorzugt vollständig, automatisiert ist.

In einer bevorzugten Ausführungsvariante kann vorgesehen sein, dass zumindest eine Steuer- und/oder Regeleinheit zur Steuerung und/oder Regelung zumindest einer der Vorbereitungsvorrichtungen vorgesehen ist.

Mit einer Steuer- und/oder Regeleinheit kann beispielsweise die Menge an Druckmedium und/oder der zu erreichende Druck innerhalb der befüllten Reinigungskomponente gesteuert und/oder geregelt werden.

In einer bevorzugten Ausführungsvariante kann vorgesehen sein, dass zumindest ein Sensor vorgesehen ist, bevorzugt wobei der Sensor mit zumindest einer Steuer- und/oder Regeleinheit verbunden ist.

Mit einem Sensor, bevorzugt einem Drucksensor, kann der Zustand der befüllten und/oder nicht-befüllten Reinigungskomponente überwachen werden.

Durch den Einsatz von zumindest teilweise, bevorzugt vollständig, automatisierten Vorbereitungsvorrichtungen wie einer Verformungsvorrichtung, einer Platziervorrichtung und/oder einer Aufbringvorrichtung, beispielsweise mit Hilfe einer Steuer- und/oder Regeleinheit und/oder mit Hilfe von Sensoren, kann die Effizienz der Reinigung der Umhausung mit der Reinigungseinheit gesteigert, die Effizienz des Betriebs der medizinischen Bildgebungsvorrichtung vergrößert und der Vorbereitungsaufwand für Vorbereitungspersonal verringert werden.

Gemäß einer bevorzugten Ausführungsform des Vorbereitungsstation ist es vorgesehen, dass die Verformungsvorrichtung einen Verformungsantrieb, ein Druckmedium und eine Verbindungsleitung zum, bevorzugt lösbaren oder unlösbaren, Verbinden der Reinigungskomponente mit dem Verformungsantrieb aufweist, wobei das Druckmedium mittels des Verformungsantriebs in die Reinigungskomponente einfüllbar und/oder aus der Reinigungskomponente entnehmbar ist, wobei die Reinigungskomponente durch Befüllen mit dem Druckmedium und/oder Entnehmen des Druckmediums verformbar ist.

Unter "lösbares Verbinden", "lösbar verbunden" und "lösbar verbindbar" kann verstanden werden, dass zumindest zwei ursprünglich voneinander getrennte Komponenten miteinander verbunden werden können, wobei dieses lösbare Verbinden anschließend zerstörungsfrei aufgelöst werden kann, sodass die zumindest zwei Komponenten wieder in ihrem ursprünglichen voneinander getrennten Zustand vorliegen, um daraufhin erneut verbunden werden zu können. "Lösbares Verbinden", "lösbar verbunden" und "lösbar verbindbar" kann daher auch als "zerstörungsfrei lösbares Verbinden", "zerstörungsfrei lösbar verbunden" und "zerstörungsfrei lösbar verbindbar" bezeichnet werden.

Unter "unlösbares Verbinden", "unlösbar verbunden" und "unlösbar verbindbar" kann verstanden werden, dass zumindest zwei ursprünglich voneinander getrennte Komponenten miteinander verbunden werden können, wobei dieses unlösbare Verbinden anschließend nicht aufgelöst werden kann, ohne dass die Integrität zumindest einer der zumindest zwei Komponenten beschädigt wird und/oder ohne dass ein erneutes Verbinden der zumindest zwei Komponenten erschwert oder ausgeschlossen wird. "Unlösbares Verbinden", "unlösbar verbunden" und "unlösbar verbindbar" kann daher auch als "zerstörungsfrei unlösbares Verbinden", "zerstörungsfrei unlösbar verbunden" und "zerstörungsfrei unlösbar verbindbar" bezeichnet werden.

In einer bevorzugten Ausführungsvariante kann vorgesehen sein, dass eine Verformungsvorrichtung einen Kompressor, bevorzugt einen Luftkompressor, aufweist, wobei der Kompressor das Druckmedium, bevorzugt Umgebungsluft, ansaugt, über eine Verbindungsleitung unter Druck zur Reinigungskomponente führt und die Reinigungskomponente somit befüllt.

Gemäß einer bevorzugten Ausführungsform des Vorbereitungsstation ist es vorgesehen, dass die Reinigungsauflage mittels der Platziervorrichtung auf den Reinigungsgrundkörper, bevorzugt entlang einer äußeren Kontur des Reinigungsgrundkörpers, platzierbar, bevorzugt aufspannbar, ist.

Durch die Platziervorrichtung kann ein Entfernen einer Reinigungsauflage von der Reinigungseinheit oder mit anderen Worten ein Deplatzieren einer Reinigungsauflage der Reinigungseinheit durchgeführt werden. In einer bevorzugten Ausführungsvariante kann somit ein Entfernen einer ersten, bevorzugt gebrauchten, Reinigungsauflage und/oder ein Platzieren einer zweiten, bevorzugt ungebrauchten, Reinigungsauflage erfolgen.

Gemäß einer bevorzugten Ausführungsform des Vorbereitungsstation ist es vorgesehen, dass die Platziervorrichtung zumindest einen Platzierantrieb und eine Verbindungsvorrichtung zum, bevorzugt lösbaren oder unlösbaren, Verbinden mit der Reinigungsauflage aufweist, wobei die mit der Verbindungsvorrichtung verbundene Reinigungsauflage durch den Platzierantrieb bis zu einer Betriebsstellung auf den Reinigungsgrundkörper bewegbar, bevorzugt von einer Rolle abwickelbar, ist.

Eine Verbindungsvorrichtung kann beispielsweise eine Klemme oder eine Achse zum Aufwickeln einer Reinigungsauflage sein.

In einer Betriebsstellung der Reinigungsauflage ist die Reinigungsauflage derart auf der Reinigungskomponente angeordnet, dass die Reinigungseinheit betriebsbereit ist und für das Reinigen der Umhausung verwendet werden kann. Bevorzugt kann vorgesehen sein, dass die Reinigungsauflage in der Betriebsstellung den Reinigungsgrundkörper teilweise oder vollständig bedeckt.

In einer bevorzugten Ausführungsvariante kann vorgesehen sein, dass Platziervorrichtung zum Aufspannen der Reinigungsauflage auf einen Reinigungsgrundkörper vorgesehen ist.

Gemäß einer bevorzugten Ausführungsform des Vorbereitungsstation ist es vorgesehen, dass das Reinigungsmittel mittels der Aufbringvorrichtung auf und/oder in die Reinigungsauflage vor, während und/oder nach einem Platzieren der Reinigungsauflage auf den Reinigungsgrundkörper, aufbringbar ist.

In einer bevorzugten Ausführungsvariante kann vorgesehen sein, dass die Aufbringvorrichtung zumindest eine Sprühdüse und/oder eine Wanne und/oder ein Reservoir und/oder eine Aufbringwalze und/oder eine Aufbringrolle zum Aufbringen des Reinigungsmittels auf die Reinigungsauflage aufweist.

In einer bevorzugten Ausführungsvariante kann vorgesehen sein, dass ein Vorratsbehälter mit einer Mehrzahl an Reinigungsauflagen vorgesehen ist, bevorzugt wobei die Reinigungsauflagen mit einem Reinigungsmittel vorbehandelt sind.

In einer bevorzugten Ausführungsvariante kann vorgesehen sein, dass die Reinigungsauflagen aus dem Vorratsbehälter mit dem Reinigungsmittel vorbefeuchtet sein können.

Die Reinigungseinheit kann manuell und/oder automatisch mit einzelnen Reinigungsauflagen aus dem Vorratsbehälter bespannt werden.

Gemäß einer bevorzugten Ausführungsform des Vorbereitungsstation ist es vorgesehen, dass eine Transfervorrichtung zum Transferieren der Reinigungseinheit zwischen der Vorbereitungsstation und einer medizinischen Bildgebungsvorrichtung vorgesehen ist, wobei Transfervorrichtung manuell antreibbar und/oder motorisiert ist.

In einer bevorzugten Ausführungsvariante kann vorgesehen sein, dass die Transfervorrichtung zumindest teilweise, bevorzugt vollständig, automatisiert ist.

Gemäß einer bevorzugten Ausführungsform des Vorbereitungsstation ist es vorgesehen, dass eine Verfahrvorrichtung vorgesehen ist, wobei die mit der Vorbereitungsstation lösbar verbundene Reinigungseinheit mittels der Verfahrvorrichtung zu der medizinischen Bildgebungsvorrichtung hin und/oder von der medizinischen Bildgebungsvorrichtung weg und/oder innerhalb der medizinischen Bildgebungsvorrichtung in zumindest eine Richtung, bevorzugt in zumindest zwei Richtungen, bewegbar ist.

Durch eine Verfahrvorrichtung kann die Reinigungseinheit aus einer Vorbereitungsstellung, bevorzugt zumindest teilweise, besonders bevorzugt vollständig automatisiert, in die Umhausung bewegt werden, dort die Reinigung der Umhausung durchführen und anschließend zurück in die Vorbereitungsstellung bewegt werden. Somit ermöglicht die Vorbereitungsstation nicht nur das Vorbereiten der Reinigungseinheit, sondern zusätzlich das Reinigen der Umhausung, wobei durch diese Kombination an Funktionalitäten eine Vereinfachung und Effizienzsteigerung der Vorbereitung der Reinigungseinheit, der Reinigung der Umhausung und des Betriebes der medizinischen Bildgebungsvorrichtung erreicht werden kann.

In einer bevorzugten Ausführungsvariante kann vorgesehen sein, dass die Verfahrvorrichtung zumindest teilweise, bevorzugt vollständig, automatisiert ist.

Bisher Gesagtes zu automatisierten Vorrichtungen und Steuer- und/oder Regeleinheiten und Sensoren gilt sinngemäß für die Transfervorrichtung und/oder die Verfahrvorrichtung.

In einer bevorzugten Ausführungsvariante kann vorgesehen sein, dass die Vorbereitungsstation als Ganzes mobil, bevorzugt über Rollen beweglich, oder feststehend ist.

In einer bevorzugten Ausführungsvariante kann vorgesehen sein, dass die Vorbereitungsstation zumindest eine Feststellvorrichtung zum Sichern der mobilen Vorbereitungsstation, besonders bevorzugt zumindest eine Bremsvorrichtung, oder zumindest eine Feststellvorrichtung zum Sichern der feststehenden Vorbereitungsstation, besonders bevorzugt zumindest einen Gurt, aufweist

Gemäß einer bevorzugten Ausführungsform des Vorbereitungsstation ist es vorgesehen, dass die Trägerstruktur formstabil ist und/oder die Reinigungskomponente elastisch verformbar ist.

Durch die elastische Verformbarkeit der Reinigungskomponente kann ein höherer Druck des Druckmediums und somit eine größere räumliche Ausdehnung erreicht werden, wodurch die Effizienz der Reinigung der Umhausung gesteigert werden kann.

In einer bevorzugten Ausführungsvariante kann vorgesehen sein, dass elektronische Bauteile zum Vorbereiten der Reinigungseinheit größtenteils, bevorzugt vollständig, an oder in der Vorbereitungsstation angeordnet sind.

In einer bevorzugten Ausführungsvariante kann vorgesehen sein, dass die Reinigungseinheit zumindest teilweise, bevorzugt vollständig, frei von metallischen und/oder elektronischen Bauteilen ist.

In einer bevorzugten Ausführungsvariante kann vorgesehen sein, dass die Trägerstruktur und/oder die Reinigungskomponente zumindest einen nichtmetallischen Werkstoff aufweist oder daraus besteht, bevorzugt wobei der zumindest eine nichtmetallische Werkstoff zumindest eines der folgenden Materialien aufweist oder daraus besteht:
- Naturwerkstoff, besonders bevorzugt Grafit,
- Polymerwerkstoff, besonders bevorzugt Elastomer, Thermoplast, Duroplast, Copolymer,
- anorganischer Werkstoff, besonders bevorzugt Keramik oder Glas,
- Verbundwerkstoff, besonders bevorzugt verstärkter Faserverbundwerkstoff oder Schichtverbundwerkstoff.

Elektronische Bauteile und/oder metallische Bauteile können die medizinische Bildgebung negativ beeinflussen und/oder durch das Magnetfeld der medizinischen Bildgebung beschädigt werden. Eine Anordnung dieser Bauteile in der Vorbereitungsstation statt in der Reinigungseinheit führt daher zu verlässlicheren Bildgebungen, Vermeiden von Beschädigungen und/oder zu einer Reduktion des Vorbereitungsaufwands.

Weiters wird Schutz begehrt für eine Anordnung mit einer hier offenbarten Vorbereitungsstation und mit einer medizinischen Bildgebungsvorrichtung, wobei die medizinische Bildgebungsvorrichtung eine Scannereinheit, einen von der Scannereinheit zumindest teilweise umgebenen Patientenaufnahmebereich und eine den Patientenaufnahmebereich umgebenden Umhausung aufweist, und mit einer Reinigungseinheit zum Reinigen der Umhausung.

In einer bevorzugten Ausführungsvariante kann vorgesehen sein, dass die Vorbereitungsstation entlang einer geschlossenen Seite der Scannereinheit oder an einer geöffneten Seite der Scannereinheit angeordnet ist.

Durch die Anordnung entlang einer geschlossenen Seite der Scannereinheit wird keine Öffnung der Umhausung blockiert, wodurch die Zugänglichkeit der Umhausung für die Bildgebung und/oder die Reinigung erhöht wird.

Durch die Anordnung entlang einer geöffneten Seite der Scannereinheit wird zwar eine Öffnung der Umhausung blockiert, jedoch kann dafür die Vorbereitungsstation im Fall einer vorhandenen Verfahrvorrichtung zum Reinigen der Umhausung in die Umhausung hinein, innerhalb der Umhausung und aus der Umhausung heraus bewegt werden. Bevorzugt kann vorgesehen sein, dass die Verfahrvorrichtung Teil der Vorbereitungsstation oder eine Verfahrvorrichtung der medizinischen Bildgebungsvorrichtung, beispielsweise ein Patiententisch, ist.

Gemäß einer bevorzugten Ausführungsform der Anordnung ist es vorgesehen, dass die Anordnung eine von der Vorbereitungsstation getrennte Verfahrvorrichtung, bevorzugt samt einem Patiententisch, aufweist, wobei die Reinigungseinheit von der Vorbereitungsstation an die getrennte Verfahrvorrichtung, bevorzugt an den Patiententisch, übergebar ist und/oder die Reinigungseinheit von der getrennten Verfahrvorrichtung, bevorzugt von dem Patiententisch, an die Vorbereitungsstation übergebar ist.

Im Fall einer getrennten Verfahrvorrichtung, bevorzugt mit einem Patiententisch, ist es vorteilhaft, dass die Vorbereitungsstation an einer geöffneten Seite der Scannereinheit angeordnet ist

In einer bevorzugten Ausführungsvariante kann vorgesehen sein, dass die Reinigungskomponente einen Reinigungsgrundkörper und eine Reinigungsauflage aufweist, wobei die Reinigungsauflage entlang einer äußeren Kontur des Reinigungsgrundkörpers platziert ist.

In einer bevorzugten Ausführungsvariante kann vorgesehen sein, dass die Reinigungskomponente entlang einer, bevorzugt äußeren, Kontur der Trägerstruktur angeordnet ist, wobei die Reinigungskomponente durch Befüllen mit einem Druckmedium verformbar ist.

Unter "durch Befüllen mit einem Druckmedium verformbare Reinigungskomponente" kann hier verstanden werden, dass eine Reinigungskomponente verformbar ist, indem ein teilweise, bevorzugt vollständig, durch die Reinigungskomponente bestimmtes Volumen zumindest teilweise, bevorzugt vollständig, durch ein Druckmedium eingenommen wird.

In einer bevorzugten Ausführungsvariante kann vorgesehen sein, dass die Reinigungskomponente der in die Umhausung eingeführten Reinigungseinheit zwischen der Trägerstruktur und der Umhausung angeordnet ist, wobei die Reinigungskomponente in einem mit einem Druckmedium befüllten Zustand an der Umhausung, bevorzugt pressend, anliegt.

In einer bevorzugten Ausführungsvariante kann vorgesehen sein, dass der Reinigungsgrundkörper ein schlauchförmiger Körper sein kann

In einer bevorzugten Ausführungsvariante kann vorgesehen sein, dass der Reinigungsgrundkörper eine flache Materialbahn, bevorzugt eine Membran, sein kann, wobei zumindest ein Ende, bevorzugt zumindest zwei Enden, der flachen Materialbahn, bevorzugt der Membran, mit der Trägerstruktur, bevorzugt entlang einer äußeren Kontur der Trägerstruktur, verbunden sein kann.

In einer bevorzugten Ausführungsvariante kann vorgesehen sein, dass der Reinigungsgrundkörper ein extrudiertes Profil und/oder ein extrudiertes Doppelprofil sein kann.

In einer bevorzugten Ausführungsvariante kann vorgesehen sein, dass die Reinigungsauflage einen Schaumstoff und/oder ein textiles Gebilde und/oder ein Nonwoven-Material aufweist.

In einer bevorzugten Ausführungsvariante kann vorgesehen sein, dass die Reinigungsauflage einen Schwamm und/oder einen Vliesstoff aufweist.

In einer bevorzugten Ausführungsvariante kann vorgesehen sein, dass die Reinigungsauflage eine Wischlippe aufweist oder daraus besteht.

In einer bevorzugten Ausführungsvariante kann vorgesehen sein, dass die Reinigungsauflage befeuchtet oder unbefeuchtet ist.

In einer bevorzugten Ausführungsvariante kann vorgesehen sein, dass eine räumliche Ausdehnung, bevorzugt ein Außendurchmesser, der Reinigungseinheit
- bei nicht-befüllter Reinigungskomponente kleiner als die räumliche Ausdehnung, bevorzugt der Außendurchmesser, der Reinigungseinheit bei befüllter Reinigungskomponente ist und/oder
- bei nicht-befüllter Reinigungskomponente kleiner als eine räumliche Ausdehnung, bevorzugt ein Innendurchmesser, der Umhausung ist und/oder
- bei befüllter Reinigungskomponente mindestens gleich groß wie oder größer als eine räumlichen Ausdehnung, bevorzugt ein Innendurchmesser, der Umhausung ist.

In einer bevorzugten Ausführungsvariante kann vorgesehen sein, dass das Druckmedium ein Gas oder eine Flüssigkeit, bevorzugt Druckluft, ist.

Ein Gas oder eine Flüssigkeit als Druckmedium können unterschiedliche Vorteile mit sich bringen. Beispielsweise sind Gase komprimierbar und können daher zu hohe Drücke besser ausgleichen. Flüssigkeiten sind kaum komprimierbar und übertragen daher Drücke nahezu ohne Druckverlust.

Weitere Einzelheiten und Vorteile bevorzugter Ausgestaltungsbeispiele der Erfindung werden anhand der Figurenbeschreibung unter Bezugnahme auf die Zeichnungen im Folgenden näher erläutert. Darin zeigen:
- Fig. 1:: eine schematische, perspektivische Ansicht einer medizinischen Bildgebungsvorrichtung;
- Fig. 2:: eine schematische, perspektivische Ansicht einer Vorbereitungsstation;
- Fig. 3 und 4:: zwei schematische, perspektivische Ansichten einer Ausführungsvariante der Reinigungseinheit;
- Fig. 5 und 6:: zwei schematische Seitenansichten zweier Ausführungsvarianten einer Anordnung mit einer Vorbereitungsstation und einer Reinigungseinheit;
- Fig. 7 bis 9:: drei schematische Seitenansichten von zwei Ausführungsvarianten einer Anordnung mit einer Vorbereitungsstation, einer Reinigungseinheit und einer medizinischen Bildgebungsvorrichtung;
- Fig. 10 und 11:: zwei schematische Seitenansichten von einer weiteren Ausführungsvarianten einer Anordnung mit einer Vorbereitungsstation, einer Reinigungseinheit und einer medizinischen Bildgebungsvorrichtung;
- Fig. 12:: eine schematische Draufsicht einer Anordnung mit einer Reinigungseinheit und einer medizinischen Bildgebungsvorrichtung und verschiedenen Positionen einer Vorbereitungsstation.

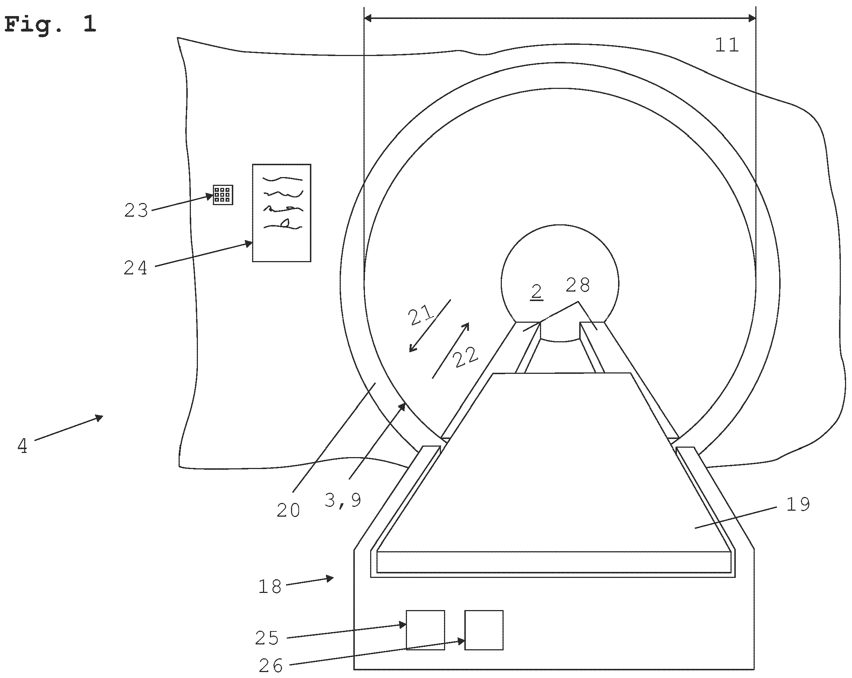

Fig. 1 zeigt eine schematische, perspektivische Ansicht einer medizinischen Bildgebungsvorrichtung 4.

Die medizinische Bildgebungsvorrichtung 4 in Fig. 1 ist eine medizinische Bildgebungsvorrichtung 4 mit einer Scannereinheit 20, einem von der Scannereinheit 20 zumindest teilweise umgebenen Patientenaufnahmebereich 2, einer den Patientenaufnahmebereich 2 umgebenden Umhausung 3.

Eine solche medizinische Bildgebungsvorrichtung 4 kann zumindest eine Reinigungseinheit 1 aufweisen, wobei die Reinigungseinheit 1 innerhalb der Umhausung 3 bewegbar sein kann.

Eine solche medizinische Bildgebungsvorrichtung 4 kann eine Bedienungsvorrichtung 23 und eine Ausgabevorrichtung 24 aufweisen.

Eine solche medizinische Bildgebungsvorrichtung 4 kann eine Verfahrvorrichtung 18 aufweisen. Die Verfahrvorrichtung 18 kann einen beweglichen Patiententisch 19, einen Verfahrantrieb 25 und eine Steuer- und Regeleinheit 26 aufweisen.

Die Verfahrvorrichtung 18 kann den Patiententisch 19 in zumindest eine Richtung 21,22, bevorzugt in eine erste Richtung 21 und in eine zweite Richtung 22, bewegen.

Die Umhausung 3 kann eine innere Kontur 9 aufweisen, wobei die innere Kontur 9 kreisförmig oder kreisbogenförmig wie in Fig. 1 sein kann.

Die Umhausung 3 und/oder eine innere Kontur 9 der Umhausung 3 kann eine räumliche Ausdehnung 11 der Umhausung 3 aufweisen.

Die medizinische Bildgebungsvorrichtung 4 kann innerhalb der Umhausung 3 Lichtleisten 28 zum Beleuchten des Patientenaufnahmebereichs 2 innerhalb der Umhausung 3 aufweisen.

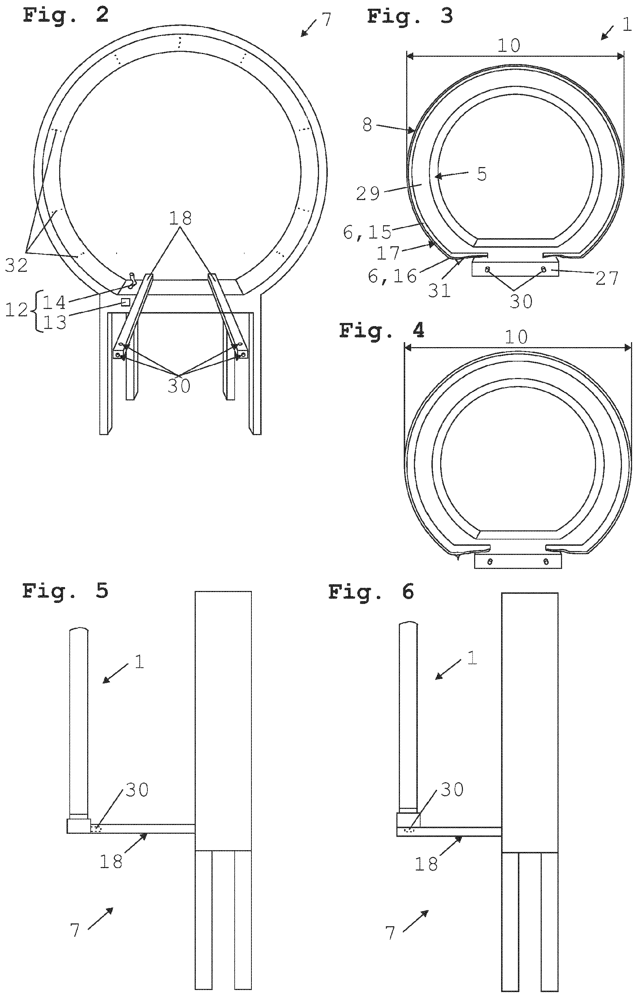

Fig. 2 zeigt eine schematische, perspektivische Ansicht einer Vorbereitungsstation 7.

Fig. 2 zeigt eine Vorbereitungsstation 7 zum Vorbereiten einer Reinigungseinheit 1, wobei die Reinigungseinheit 1 zum Reinigen einer einen Patientenaufnahmebereich 2 umgebenden Umhausung 3 einer medizinischen Bildgebungsvorrichtung 4 vorgesehen ist, wobei die Reinigungseinheit 1 eine Trägerstruktur 5 und eine Reinigungskomponente 6 aufweist, wobei die Vorbereitungsstation 7 von der medizinischen Bildgebungsvorrichtung 4 getrennt und in der Nachbarschaft derselben angeordnet ist, wobei die Vorbereitungsstation 7 zumindest eine Vorbereitungsvorrichtung aufweist, durch welche die Reinigungseinheit 1 für eine nachfolgende Reinigung vorbereitet wird.

Die Vorbereitungsstation 7 in Fig. 2 weist einen im Wesentlichen ringförmigen, konkret einen kreisbogenförmigen, oberen Teil und einen unteren Tisch, auf dem der obere Teil angeordnet ist, auf.

Innerhalb des oberen Teils können entlang der inneren Kontur des oberen Teils Düsen zum Aufbringen eines Reinigungsmittels angeordnet sein. Diese Düsen stellen eine Aufbringvorrichtung 32 dar, wobei aus Gründen der Übersichtlichkeit nicht jede Düse in Fig. 2 einzeln mit einem Bezugszeichen beschriftet ist. Befindet sich eine Reinigungseinheit 1 innerhalb des oberen Teils oder, mit anderen Worten, wenn eine Reinigungseinheit 1 derart angeordnet ist, sodass die Reinigungseinheit 1 von der Vorbereitungsstation 7, konkret der Aufbringvorrichtung 32, umgeben ist, dann kann ein Reinigungsmittel auf die Reinigungskomponente 6 der Reinigungseinheit 1 aufgebracht werden.

Die Vorbereitungsstation 7 in Fig. 2 weist eine Verformungsvorrichtung 12 zum Verformen der Reinigungskomponente 6 auf, wobei die Verformungsvorrichtung 12 einen Verformungsantrieb 13, ein Druckmedium und eine Verbindungsleitung 14 zum, bevorzugt lösbaren oder unlösbaren, Verbinden der Reinigungskomponente 6 mit dem Verformungsantrieb 13 aufweist, wobei das Druckmedium mittels des Verformungsantriebs 13 in die Reinigungskomponente 6 einfüllbar und/oder aus der Reinigungskomponente 6 entnehmbar ist, wobei die Reinigungskomponente 6 durch Befüllen mit dem Druckmedium und/oder Entnehmen des Druckmediums verformbar ist.

Der Verformungsantrieb 13 kann beispielsweise ein Luftkompressor sein, welcher Luft aus der Umgebung durch die Verbindungsleitung 14 fördert und somit die Reinigungskomponente 6 mit Druckluft befüllt.

Die Vorbereitungsstation 7 in Fig. 2 weist eine Verfahrvorrichtung 18 mit zwei Verfahrschienen auf, wobei auf beiden Verfahrschienen jeweils eine Kopplungsvorrichtung 30 zum lösbaren Verbinden der Reinigungseinheit 1 mit der Vorbereitungsstation 7 auf der Vorderseite und auf der Oberseite der Verfahrschienen vorgesehen ist. Somit kann eine Reinigungseinheit 1 an der Vorderseite und/oder an der Oberseite der Verfahrschienen angeordnet werden.

Fig. 3 zeigt eine schematische, perspektivische Ansicht einer Ausführungsvariante der Reinigungseinheit 1 im nicht-befüllten Zustand.

Die Reinigungseinheit 1 kann eine Trägerstruktur 5 und eine Reinigungskomponente 6 aufweisen.

Die Trägerstruktur 5 kann ein Basisteil 27 und ein Ringteil 29 aufweisen, wobei das Basisteil 27 zumindest eine Kopplungsvorrichtung 30 zum lösbaren Verbinden mit einer Verfahrvorrichtung 18 aufweisen kann.

Die Position der Kopplungsvorrichtungen 30 kann auf dem Basisteil 27 und/oder auf dem Ringteil 29 vorgesehen sein. Für die Fälle, dass die Verfahrvorrichtung 18 zum Bewegen der Reinigungseinheit 1 einen Patiententisch 19 aufweist und/oder dass die Verfahrvorrichtung 18 eine von der medizinischen Bildgebungsvorrichtung getrennte Vorrichtung ist, können unterschiedliche Positionen der zumindest einen Kopplungsvorrichtung 30 vorteilhaft sein.

Die Anzahl und Positionen zumindest einer Kopplungsvorrichtung 30 kann nach Bedarf vorgesehen sein. Die dargestellte Ausführungsvariante ist nicht limitierend zu verstehen.

Das Ringteil 29 der Trägerstruktur 5 in Fig. 3 und 4 weist eine äußere Kontur auf, welche nahezu einen vollständigen Kreis, konkret einen Kreisbogen mit einem Winkel über 180°, beschreibt. Es kann vorgesehen sein, dass die Trägerstruktur 5 eine äußere Kontur aufweist, welche einen vollständigen Kreis oder einen unvollständigen Kreis, konkret einen Kreisbogen, aufweist.

Die Reinigungskomponente 6 kann einen Reinigungsgrundkörper 15 und eine Reinigungsauflage 16 aufweisen, wobei der Reinigungsgrundkörper 15 zwischen der Trägerstruktur 5, konkret dem Ringteil 29 und/oder der äußeren Kontur der Trägerstruktur 5, und der Reinigungsauflage 16 angeordnet sein kann.

Es kann vorgesehen sein, dass die Reinigungskomponente 6 über den Kreisbogen des Ringteils 29 hinaus bis zum Basisteil 27 weiterverläuft. Auf diese Weise kann sowohl die Innenseite der Umhausung 3 als auch zumindest eine Seite der Lichtleisten 28, konkret die Oberseite der Lichtleiste 28, gereinigt werden. Mit Hilfe der Ausführungsvariante aus Fig. 3 und 4 überdeckt die Reinigungskomponente 6 auch den Spalt, der zwischen den Lichtleisten 28 und dem Basisteil 27 ist.

Die Reinigungsauflage 16 kann flexibel ausgeführt sein, sodass sich die Reinigungsauflage 16 der Form des Reinigungsgrundkörpers 15 anpasst. In Fig. 3 und 4 entspricht die Form einer äußeren Kontur des Reinigungsgrundkörpers 15 im Wesentlichen der Form der äußeren Kontur der auf dem Reinigungsgrundkörper 15 angeordneten Reinigungsauflage 16.

In Fig. 3 und 4 entspricht die Form einer äußeren Kontur der Trägerstruktur 5 im Wesentlichen der Form der äußeren Kontur 8 der Reinigungskomponente 6, konkret in Fig. 3 und 4 der äußeren Kontur der auf der Reinigungskomponente 6 angeordneten Reinigungsauflage 16.

In Fig. 3 und 4 ist die Form der äußeren Kontur 8 der Reinigungskomponente 6 im Wesentlichen kreisförmig, konkret kreisbogenförmig.

Zum, bevorzugt lösbaren oder unlösbaren, Verbinden der Reinigungskomponente 6 mit der Verbindungsleitung 14 kann die Reinigungskomponente 6 einen Anschluss 31 aufweisen. Der Anschluss kann verschließbar sein und entweder manuell oder automatisch geöffnet und/oder geschlossen werden.

Fig. 4 zeigt eine schematische, perspektivische Ansicht einer Ausführungsvariante der Reinigungseinheit 1 im befüllten Zustand.

Bisher Gesagtes zu Fig. 3 gilt analog für Fig. 4.

In Fig. 3 weist die Reinigungskomponente 6 kein, also einen nicht-befüllten Zustand, auf. Wichtig ist, dass die äußere Kontur 8 der Reinigungskomponente 6 eine räumliche Ausdehnung 10 aufweist, welche kleiner als eine räumliche Ausdehnung 10 der Reinigungskomponente 6 im befüllten Zustand und/oder kleiner als eine räumliche Ausdehnung 11 der Umhausung 3 ist. Mit anderen Worten, wichtig ist, dass die äußere Kontur 8 der Reinigungskomponente 6 im nicht-befüllten Zustand die Umhausung nicht kontaktiert.

In Fig. 4 ist klar erkenntlich, dass die räumliche Ausdehnung 10 der Reinigungskomponente 6 grösser als die räumlichen Ausdehnung 10 der Reinigungskomponente 6 in Fig. 3 ist. Dieser befüllte Zustand der Reinigungskomponente 6 kann durch Befüllen der Reinigungskomponente 6 mit einem Druckmedium durch die Verformungsvorrichtung 12 erreicht werden.

Es sei an dieser Stelle angemerkt, dass die Reinigungseinheit 1 zumindest zwei gleichartige Reinigungskomponenten 6 oder zumindest zwei verschiedenartige aufweisen kann.

Fig. 5 zeigt eine schematische Seitenansicht einer Ausführungsvariante einer Anordnung mit einer Vorbereitungsstation 7 und einer Reinigungseinheit 1, wobei die Reinigungseinheit 1 an der Vorderseite der Verfahrvorrichtung 18, konkret den Verfahrschienen, angeordnet ist und sich wie in Fig. 3 in einem nicht-befüllten Zustand befindet.

In Fig. 5 und allen nachfolgenden Fig. 6 bis 10 deuten strichlierte Linien verdeckte Konstruktionsmerkmale an. So sind beispielsweise in Fig. 5 und 6 die Kopplungsvorrichtungen 30 strichliert dargestellt, weil sie in Anbetracht der Ausführungsvariante in Fig. 2 in der Seitenansicht in Fig. 5 und 6 von außen nicht erkenntlich sind.

Fig. 6 zeigt eine schematische Seitenansicht einer Ausführungsvariante einer Anordnung mit einer Vorbereitungsstation 7 und einer Reinigungseinheit 1, wobei die Reinigungseinheit 1 an der Oberseite der Verfahrvorrichtung 18, konkret den Verfahrschienen, angeordnet ist und sich wie in Fig. 3 in einem nicht-befüllten Zustand befindet.

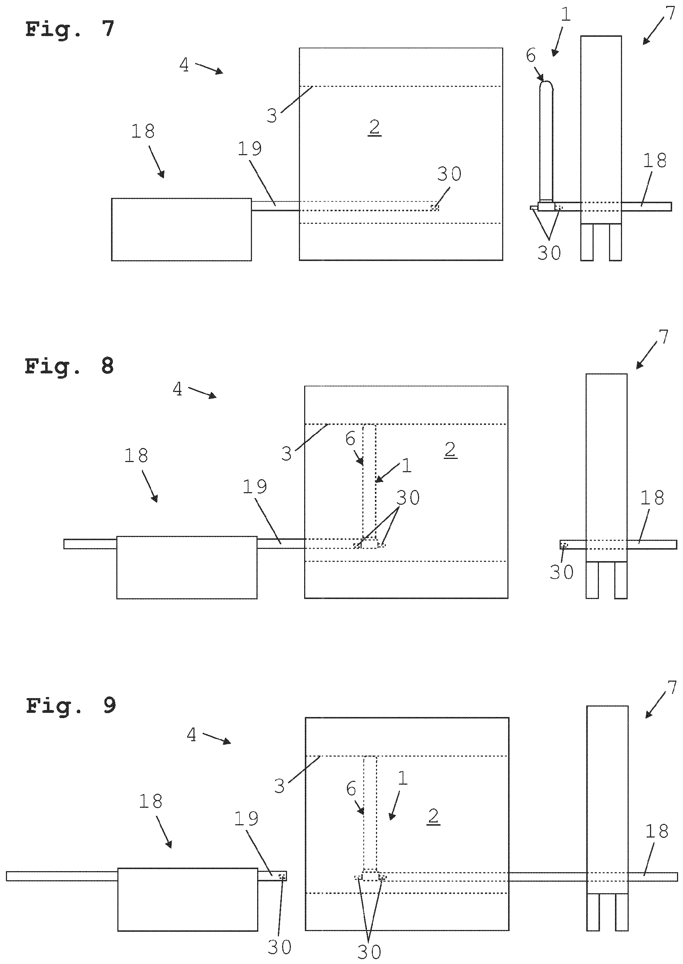

Fig. 7 zeigt eine schematische Seitenansicht einer Ausführungsvariante einer Anordnung mit einer Vorbereitungsstation 7, einer Reinigungseinheit 1, welche sich wie in Fig. 4 in einem befüllten Zustand befindet, und einer medizinischen Bildgebungsvorrichtung 4.

Fig. 7 zeigt eine Anordnung mit einer erfindungsgemäßen Vorbereitungsstation 7 und mit einer medizinischen Bildgebungsvorrichtung 4, wobei die medizinische Bildgebungsvorrichtung 4 eine Scannereinheit 20, einen von der Scannereinheit 20 zumindest teilweise umgebenen Patientenaufnahmebereich 2 und eine den Patientenaufnahmebereich 2 umgebenden Umhausung 3 aufweist, und mit einer Reinigungseinheit 1 zum Reinigen der Umhausung 3. Zusätzlich ist eine von der Vorbereitungsstation 7 und der medizinischen Bildgebungsvorrichtung 4 getrennte Verfahrvorrichtung 18 vorgesehen.

Der Patiententisch 19 der getrennten Verfahrvorrichtung 18 in Fig. 7 links neben der medizinischen Bildgebungsvorrichtung 4 ist so weit ausgefahren, dass ein Teil des Patiententisches 19 im Patientenaufnahmebereich 2 innerhalb der Umhausung 3 ist. Die Reinigungseinheit 1 ist, wie in Fig. 5 dargestellt, mit der Vorbereitungsstation 7 lösbar verbunden.

In Fig. 7 kann die Verfahrvorrichtung 18 der Vorbereitungsstation 7 die Reinigungseinheit 1 in die Umhausung 3 hineinbewegen und/oder die Reinigungseinheit 1 aus der Umhausung 3 hinausbewegen.

Fig. 8 zeigt die Anordnung aus Fig. 7, wobei eine Übergabe, bevorzugt innerhalb oder außerhalb der Umhausung 3, der Reinigungseinheit 1 von der Vorbereitungsstation 7 an den Patiententisch 19 der getrennten Verfahrvorrichtung 18 stattgefunden hat. Es kann eine entsprechende Rückübergabe vorgesehen sein. Auf diese Weise kann die Reinigungseinheit 1 zum Reinigen der Umhausung 3 mittels des Patiententisches 19 verwendet werden. Für eine Übergabe der Reinigungseinheit 1 sind Kopplungsvorrichtungen 30 an der getrennten Verfahrvorrichtung 18, der Reinigungseinheit 1 und der Verfahrvorrichtung 18 der Vorbereitungsstation 7 vorgesehen.

Im Vergleich zu Fig. 7 kann anhand der Reinigungseinheit 1, konkret der Reinigungskomponente 6, gut erkannt werden, dass die Reinigungseinheit 1, konkret die Reinigungskomponente 6 im befüllten Zustand, in Fig. 8 gegen die Umhausung 3 gepresst wird.

Fig. 9 zeigt eine schematische Seitenansicht einer weiteren Ausführungsvariante einer Anordnung mit einer Vorbereitungsstation 7, einer Reinigungseinheit 1, welche sich wie in Fig. 4 in einem befüllten Zustand befindet, und einer medizinischen Bildgebungsvorrichtung 4.

Bei der Ausführungsvariante in Fig. 9 ist die Verfahrvorrichtung 18 der Vorbereitungsstation 7 derart dimensioniert, dass die Verfahrvorrichtung 18 der Vorbereitungsstation 7 neben einem Verfahren zum Vorbereiten der Reinigungseinheit 1 auch ein Verfahren zum Reinigen der Umhausung 3 durchführen kann, da die Verfahrschienen ausreichend lang ausgeführt sind.

In Fig. 9 ist erkennbar, dass die Reinigungseinheit 1 mittels der Verfahrvorrichtung 18 der Vorbereitungsstation 7 innerhalb der Umhausung 3 von der ersten Öffnung bis zur zweiten Öffnung bewegt werden kann, um die gesamte Länge der Umhausung 3 zu reinigen.

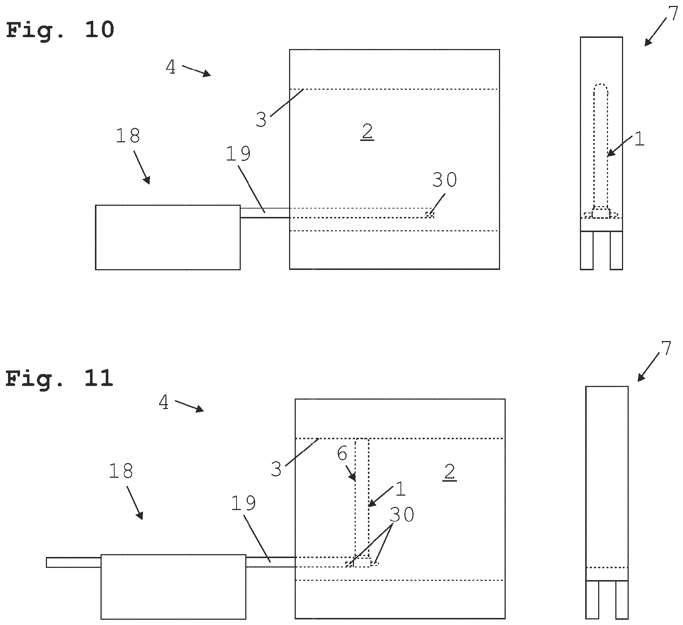

Fig. 10 und 11 zeigen eine Anordnung mit einer erfindungsgemäßen Vorbereitungsstation 7 und mit einer medizinischen Bildgebungsvorrichtung 4, wobei die medizinische Bildgebungsvorrichtung 4 eine Scannereinheit 20, einen von der Scannereinheit 20 zumindest teilweise umgebenen Patientenaufnahmebereich 2 und eine den Patientenaufnahmebereich 2 umgebenden Umhausung 3 aufweist, und mit einer Reinigungseinheit 1 zum Reinigen der Umhausung 3. Zusätzlich ist eine von der Vorbereitungsstation 7 und der medizinischen Bildgebungsvorrichtung 4 getrennte Verfahrvorrichtung 18 vorgesehen.

Im Unterschied zu den Ausführungsvarianten in Fig. 7 bis 9 weist die Vorbereitungsstation 7 keine eigene Verfahrvorrichtung 18 auf. Das Verfahren der Reinigungseinheit 1 erfolgt in diesem Ausführungsbeispiel ausschließlich mit der von der medizinischen Bildgebungsvorrichtung 4 und der von der Vorbereitungsstation 7 getrennten Verfahrvorrichtung 18 samt dazugehörigen Patiententisch 19.

Die Übergabe der Reinigungseinheit 1 in dem Ausführungsbeispiel in Fig. 10 und 11 erfolgt innerhalb der Vorbereitungsstation 7, in der die Reinigungseinheit wie in Fig. 10 angeordnet, bevorzugt geparkt und/oder vorbereitet, ist. Der Patiententisch kann bis zur Vorbereitungsstation 7 verfahren werden, anschließend die Reinigungseinheit 1 übernehmen und aus der Vorbereitungsstation 7 heraus, in die Umhausung 3 hinein und innerhalb der Umhausung 3 in zumindest eine Richtung 21,22 bewegen.

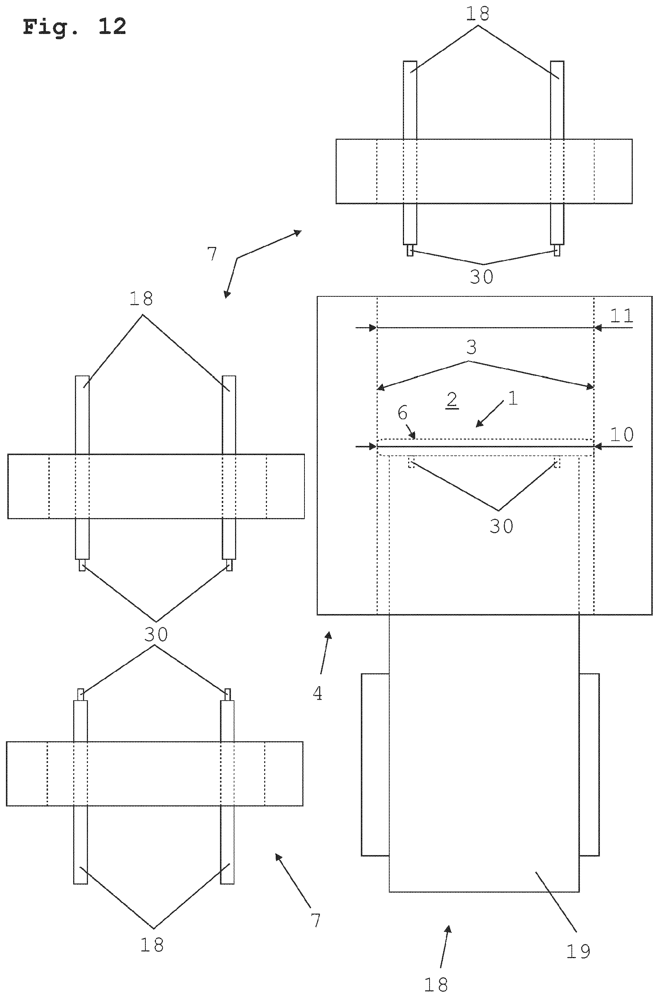

Fig. 12 zeigt eine schematische Draufsicht einer Ausführungsvariante einer Anordnung mit einer Vorbereitungsstation 7, einer Reinigungseinheit 1, welche sich wie in Fig. 4 in einem befüllten Zustand befindet, und einer medizinischen Bildgebungsvorrichtung 4. Dabei ist die Vorbereitungsstation 7 an drei verschiedenen Positionen dargestellt. Es ist nicht zwingend vorgesehen, dass drei Vorbereitungsstationen 7 vorhanden sind, jedoch soll Fig. 12 verschiedene Möglichkeiten der Positionierung der Vorbereitungsstation 7 exemplarisch verdeutlichen. Nichtsdestotrotz können auch mehr als eine Vorbereitungsstation 7 für eine derartige Anordnung vorgesehen sein, beispielsweise vorteilhaft, wenn mehr als eine Reinigungseinheit 1 zum Einsatz kommen soll.

Es kann, wie in Fig. 12 dargestellt, vorgesehen sein, dass die Vorbereitungsstation 7 entlang einer geschlossenen Seite, in Fig. 12 links neben, der medizinischen Bildgebungsvorrichtung 4, konkret der Scannereinheit 20, oder an einer geöffneten Seite, in Fig. 12 oberhalb, der medizinischen Bildgebungsvorrichtung 4, konkret der Scannereinheit 20, angeordnet ist.

Es kann auch vorgesehen sein, dass die Vorbereitungsstation 7 entlang einer geschlossenen Seite, in Fig. 12 bei der linken untern Ecke, der medizinischen Bildgebungsvorrichtung 4, konkret der Scannereinheit 20, angeordnet ist.

Wenn eine Vorbereitungsstation 7 entlang einer geschlossenen Seite der medizinischen Bildgebungsvorrichtung 4, konkret der Scannereinheit 20, angeordnet ist, kann die Reinigungseinheit über einen kurzen Weg händisch und/oder mittels einer Transfervorrichtung, bevorzugt teilweise, besonders bevorzugt vollständig, automatisch, zwischen der Vorbereitungsstation 7 und der medizinischen Bildgebungsvorrichtung 4 hin und her transferiert werden.

Wenn eine Vorbereitungsstation 7 entlang einer geöffneten Seite der medizinischen Bildgebungsvorrichtung 4, konkret der Scannereinheit 20, angeordnet ist, kann die Reinigungseinheit über einen kurzen Weg händisch und/oder mittels einer Transfervorrichtung, bevorzugt teilweise, besonders bevorzugt vollständig, automatisch, und/oder mittels zumindest einer Verfahrvorrichtung 18, bevorzugt teilweise, besonders bevorzugt vollständig, automatisch, zwischen der Vorbereitungsstation 7 und der medizinischen Bildgebungsvorrichtung 4 hin und her transferiert werden.

Im Fall einer Ausführungsvariante mit zumindest einer Verfahrvorrichtung 18, bevorzugt zumindest zwei Verfahrvorrichtungen 18 wie in Fig. 7 bis 9 dargestellt, kann es vorteilhaft sein, wenn die Vorbereitungsstation 7 an einer Öffnung der medizinischen Bildgebungsvorrichtung 4, konkret der Scannereinheit 20, angeordnet ist. Besonders bevorzugt kann vorgesehen sein, wie in Fig. 12 dargestellt, dass die Vorbereitungsstation 7 bei jener Öffnung der medizinischen Bildgebungsvorrichtung 4, konkret der Scannereinheit 20, angeordnet ist, welche einer anderen Öffnung der medizinischen Bildgebungsvorrichtung 4, konkret der Scannereinheit 20, gegenüberliegt, wobei bei der anderen Öffnung die getrennten Verfahrvorrichtung 18 samt Patiententisch 19 angeordnet ist.

In Fig. 12 kann wie in Fig. 8 und 9 anhand der Reinigungseinheit 1, konkret der Reinigungskomponente 6, gut erkannt werden, dass die Reinigungseinheit 1, konkret die Reinigungskomponente 6, gegen die Umhausung 3 gepresst wird. Dadurch entspricht die räumliche Ausdehnung 10 der Reinigungseinheit 1 der räumliche Ausdehnung 11 der Umhausung 3, weil die Umhausung 3 die räumliche Ausdehnung 10 der mit dem Druckmedium befüllten Reinigungskomponente 6, mit anderen Worten der Reinigungskomponente 6 im befüllten Zustand, begrenzt.

### Bezugszeichenliste

| | | | |
|---|---|---|---|
| 1 | Reinigungseinheit | 26 | Steuer- und Regeleinheit der Verfahrvorrichtung |
| 2 | Patientenaufnahmebereich | | |
| 3 | Umhausung | 27 | Basisteil |
| 4 | medizinische Bildgebungsvorrichtung | 28 | Lichtleiste |
| | | 29 | Ringteil |
| 5 | Trägerstruktur | 30 | Kopplungsvorrichtung |
| 6 | Reinigungskomponente | 31 | Anschluss |
| 7 | Vorbereitungsstation | 32 | Aufbringvorrichtung |
| 8 | äußere Kontur der Reinigungskomponente | | |
| 9 | innere Kontur der Umhausung | | |
| 10 | räumliche Ausdehnung der Reinigungseinheit | | |
| 11 | räumliche Ausdehnung der Umhausung | | |
| 12 | Verformungsvorrichtung | | |
| 13 | Verformungsantrieb | | |
| 14 | Verbindungsleitung | | |
| 15 | Reinigungsgrundkörper | | |
| 16 | Reinigungsauflage | | |
| 17 | äußere Kontur der Reinigungsgrundkörper | | |
| 18 | Verfahrvorrichtung | | |
| 19 | Patiententisch | | |
| 20 | Scannereinheit | | |
| 21 | erste Richtung | | |
| 22 | zweite Richtung | | |
| 23 | Bedienungsvorrichtung | | |
| 24 | Ausgabevorrichtung | | |
| 25 | Verfahrantrieb | | |

## Patentansprüche

1. Verfahren zum Vorbereiten einer Reinigungseinheit (1) und zum Reinigen einer einen Patientenaufnahmebereich (2) umgebenden Umhausung (3) einer medizinischen Bildgebungsvorrichtung (4)
- mittels der Reinigungseinheit (1), wobei die Reinigungseinheit (1) eine Trägerstruktur (5) und eine Reinigungskomponente (6) aufweist, und
- mittels einer, bevorzugt von der medizinischen Bildgebungsvorrichtung (4) getrennten und in der Nachbarschaft derselben angeordneten, Vorbereitungsstation (7) mit zumindest einer Vorbereitungsvorrichtung und mit zumindest einer Verfahrvorrichtung (18), wobei die Reinigungseinheit (1) mit der Vorbereitungsstation (7) lösbar verbunden ist,
∘ wobei zumindest ein Vorbereitungsschritt durch die zumindest eine Vorbereitungsvorrichtung durchgeführt wird, durch welchen die Reinigungseinheit (1) für eine nachfolgende Reinigung vorbereitet wird und
∘ wobei die Reinigung durch ein Bewegen der vorbereiteten Reinigungseinheit (1) innerhalb der medizinischen Bildgebungsvorrichtung (4) in zumindest eine Richtung (21,22), bevorzugt in zumindest zwei Richtungen, erfolgt.

2. Verfahren nach Anspruch 1, wobei
- die Reinigungseinheit (1) zu der Vorbereitungsstation (7) hinbewegt wird und
- anschließend zumindest ein Vorbereitungsschritt durchgeführt wird, durch welchen die Reinigungseinheit (1) für eine nachfolgende Reinigung vorbereitet wird.

3. Verfahren nach Anspruch 1 oder 2 mit zumindest einen der folgenden Verfahrensschritte:
- lösbares Verbinden der Reinigungseinheit (1) mit der Vorbereitungsstation (7), bevorzugt mittels einer Kopplungsvorrichtung (30),
- Aufladen eines Kraftspeichers und/oder eines Energiespeichers,
- Verformen, bevorzugt Aufblasen, der Reinigungskomponente (6) durch Befüllen mit einem Druckmedium oder durch Entnahme des Druckmediums,
- Platzieren, bevorzugt Aufspannen, einer Reinigungsauflage (16) der Reinigungskomponente (6) auf einen Reinigungsgrundkörper (15) der Reinigungskomponente (6), bevorzugt entlang einer äußeren Kontur (17) des Reinigungsgrundkörpers (15),
- Aufbringen und/oder Einbringen eines Reinigungsmittels auf und/oder in eine Reinigungsauflage (16) der Reinigungskomponente (6), bevorzugt vor, während und/oder nach einem Platzieren der Reinigungsauflage (16) der Reinigungskomponente (6) auf einen Reinigungsgrundkörper (15) der Reinigungskomponente (6).

4. Verfahren nach einem der vorangegangenen Ansprüche mit zumindest einem der folgenden Verfahrensschritte:
- Lösen der mit der Vorbereitungsstation (7) lösbar verbundenen Reinigungseinheit (1), bevorzugt mittels der Kopplungsvorrichtung (30),
- Transferieren der Reinigungseinheit (1) zwischen der medizinischen Bildgebungsvorrichtung (4) und der Vorbereitungsstation (7),
- Lösbares Verbinden der Reinigungseinheit (1) mit der medizinischen Bildgebungsvorrichtung (4),
- Lösen der Reinigungseinheit (1) von der medizinischen Bildgebungsvorrichtung (4).

5. Vorbereitungsstation (7) zum Vorbereiten einer Reinigungseinheit (1), wobei die Reinigungseinheit (1) zum Reinigen einer einen Patientenaufnahmebereich (2) umgebenden Umhausung (3) einer medizinischen Bildgebungsvorrichtung (4) vorgesehen ist, wobei die Reinigungseinheit (1) eine Trägerstruktur (5) und eine Reinigungskomponente (6) aufweist, wobei die Vorbereitungsstation (7) von der medizinischen Bildgebungsvorrichtung (4) getrennt und in der Nachbarschaft derselben angeordnet ist, wobei die Vorbereitungsstation (7) zumindest eine Vorbereitungsvorrichtung aufweist, durch welche die Reinigungseinheit (1) für eine nachfolgende Reinigung vorbereitet wird.

6. Vorbereitungsstation (7) nach Anspruch 5, wobei die zumindest eine Vorbereitungsvorrichtung
- eine Kopplungsvorrichtung (30) zum lösbaren Verbinden der Reinigungseinheit (1) mit der Vorbereitungsstation (7),
- eine Ladevorrichtung zum Aufladen eines Kraftspeichers und/oder eines Energiespeichers,
- eine Verformungsvorrichtung (12) zum Verformen der Reinigungskomponente (6),
- eine Platziervorrichtung zum Platzieren einer Reinigungsauflage (16) der Reinigungskomponente (6) auf einen Reinigungsgrundkörper (15) der Reinigungskomponente (6) oder
- eine Aufbringvorrichtung (32) zum Aufbringen eines Reinigungsmittels auf und/oder in eine Reinigungsauflage (16) der Reinigungskomponente (6) ist.

7. Vorbereitungsstation (7) nach Anspruch 6, wobei die Verformungsvorrichtung (12) einen Verformungsantrieb (13), ein Druckmedium und eine Verbindungsleitung (14) zum, bevorzugt lösbaren oder unlösbaren, Verbinden der Reinigungskomponente (6) mit dem Verformungsantrieb (13) aufweist, wobei das Druckmedium mittels des Verformungsantriebs (13) in die Reinigungskomponente (6) einfüllbar und/oder aus der Reinigungskomponente (6) entnehmbar ist, wobei die Reinigungskomponente (6) durch Befüllen mit dem Druckmedium und/oder Entnehmen des Druckmediums verformbar ist.

8. Vorbereitungsstation (7) nach einem der vorangegangenen Ansprüche 6 oder 7, wobei die Reinigungsauflage (16) mittels der Platziervorrichtung auf den Reinigungsgrundkörper (15), bevorzugt entlang einer äußeren Kontur (17) des Reinigungsgrundkörpers (15), platzierbar, bevorzugt aufspannbar, ist.

9. Vorbereitungsstation (7) nach einem der vorangegangenen Ansprüche 6 bis 8, wobei die Platziervorrichtung einen Platzierantrieb und eine Verbindungsvorrichtung zum, bevorzugt lösbaren oder unlösbaren, Verbinden mit der Reinigungsauflage (16) aufweist, wobei die mit der Verbindungsvorrichtung verbundene Reinigungsauflage (16) durch den Platzierantrieb bis zu einer Betriebsstellung auf den Reinigungsgrundkörper (15) bewegbar, bevorzugt von einer Rolle abwickelbar, ist.

10. Vorbereitungsstation (7) nach einem der vorangegangenen Ansprüche 6 bis 9, wobei das Reinigungsmittel mittels der Aufbringvorrichtung (32) auf und/oder in die Reinigungsauflage (16) vor, während und/oder nach einem Platzieren der Reinigungsauflage (16) auf den Reinigungsgrundkörper (15), aufbringbar ist.

11. Vorbereitungsstation (7) nach einem der vorangegangenen Ansprüche 5 bis 10, wobei eine Transfervorrichtung zum Transferieren der Reinigungseinheit (1) zwischen der Vorbereitungsstation (7) und einer medizinischen Bildgebungsvorrichtung (4) vorgesehen ist, wobei Transfervorrichtung manuell antreibbar und/oder motorisiert ist.

12. Vorbereitungsstation (7) nach einem der vorangegangenen Ansprüche 5 bis 11, wobei eine Verfahrvorrichtung (18) vorgesehen ist, wobei die mit der Vorbereitungsstation (7) lösbar verbundene Reinigungseinheit (1) mittels der Verfahrvorrichtung (18) zu der medizinischen Bildgebungsvorrichtung (4) hin und/oder von der medizinischen Bildgebungsvorrichtung (4) weg und/oder innerhalb der medizinischen Bildgebungsvorrichtung (4) in zumindest eine Richtung (21,22), bevorzugt in zumindest zwei Richtungen, bewegbar ist.

13. Vorbereitungsstation (7) nach einem der vorangegangenen Ansprüche 5 bis 12, wobei die Trägerstruktur (5) formstabil ist und/oder die Reinigungskomponente (6) elastisch verformbar ist.

14. Anordnung mit einer Vorbereitungsstation (7) nach einem der vorangegangenen Ansprüche 5 bis 13 und mit einer medizinischen Bildgebungsvorrichtung (4), wobei die medizinische Bildgebungsvorrichtung (4) eine Scannereinheit (20), einen von der Scannereinheit (20) zumindest teilweise umgebenen Patientenaufnahmebereich (2) und eine den Patientenaufnahmebereich (2) umgebenden Umhausung (3) aufweist, und mit einer Reinigungseinheit (1) zum Reinigen der Umhausung (3).

15. Anordnung nach Anspruch 14, mit einer von der Vorbereitungsstation (7) getrennten Verfahrvorrichtung (18), bevorzugt samt einem Patiententisch (19), wobei die Reinigungseinheit (1) von der Vorbereitungsstation (7) an die getrennte Verfahrvorrichtung (18), bevorzugt an den Patiententisch (19), übergebar ist und/oder die Reinigungseinheit (1) von der getrennten Verfahrvorrichtung (18), bevorzugt von dem Patiententisch (19), an die Vorbereitungsstation (7) übergebar ist.

## Claims

1. Method for preparing a cleaning unit (1) and for cleaning a housing (3) of a medical imaging device (4) that surrounds a patient-accommodating region (2)
- by means of a cleaning unit (1), wherein the cleaning unit (1) comprises a support structure (5) and a cleaning component (6), and
- by means of a preparation station (7), preferably separate from the medical imaging device (4) and arranged in the vicinity of same, having at least one preparation device and having at least one displacement device (18), wherein the cleaning unit (1) is detachably connected to the preparation station (7),
∘ wherein at least one preparation step is carried out by the at least one preparation device, by means of which the cleaning unit (1) is prepared for subsequent cleaning, and
∘ wherein cleaning is implemented by moving the prepared cleaning unit (1) within the medical imaging device (4) in at least one direction (21, 22), preferably in at least two directions.

2. Method according to claim 1, wherein
- the cleaning unit (1) is moved toward the preparation station (7) and
- at least one preparation step is subsequently carried out, by means of which the cleaning unit (1) is prepared for subsequent cleaning.

3. Method according to claim 1 or 2, including at least one of the following method steps:
- detachably connecting the cleaning unit (1) to the preparation station (7), preferably by means of a coupling device (30),
- charging a force accumulator and/or an energy accumulator,
- deforming, preferably inflating, the cleaning component (6) by filling with a pressure medium or by removing the pressure medium,
- placing, preferably stretching, a cleaning covering (16) of the cleaning component (6) on a cleaning main body (15) of the cleaning component (6), preferably along an outer contour (17) of the cleaning main body (15),
- applying and/or introducing a cleaning product onto and/or into a cleaning covering (16) of the cleaning component (6), preferably before, during and/or after placement of the cleaning covering (16) of the cleaning component (6) on a cleaning main body (15) of the cleaning component (6).

4. Method according to one of preceding claims, including at least one of the following method steps:
- detaching the cleaning unit (1) detachably connected to the preparation station (7), preferably by means of the coupling device (30),
- transferring the cleaning unit (1) between the medical imaging device (4) and the preparation station (7),
- detachably connecting the cleaning unit (1) to the medical imaging device (4),
- detaching the cleaning unit (1) from the medical imaging device (4).

5. Preparation station (7) for preparing a cleaning unit (1), wherein the cleaning unit (1) is provided to clean a housing (3) of a medical imaging device (4) that surrounds a patient-accommodating region (2), wherein the cleaning unit (1) comprises a support structure (5) and a cleaning component (6), wherein the preparation station (7) is separate from the medical imaging device (4) and arranged in the vicinity of same, wherein the preparation station (7) comprises at least one preparation device, by means of which the cleaning unit (1) is prepared for subsequent cleaning.

6. Preparation station (7) according to claim 5, wherein the at least one preparation device is
- a coupling device (30) for detachably connecting the cleaning unit (1) to the preparation station (7),
- a charging device for charging a force accumulator and/or an energy accumulator,
- a deformation device (12) for deforming the cleaning component (6),
- a placement device for placing a cleaning covering (16) of the cleaning component (6) on a cleaning main body (15) of the cleaning component (6) or
- an application device (32) for applying a cleaning product onto and/or into a cleaning covering (16) of the cleaning component (6).

7. Preparation station (7) according to claim 6, wherein the deformation device (12) comprises a deformation drive (13), a pressure medium and a connecting line (14) for, preferably detachably or non-detachably, connecting the cleaning component (6) to the deformation drive (13), wherein the pressure medium can be filled into the cleaning component (6) and/or removed from the cleaning component (6) by means of the deformation drive (13), wherein the cleaning component (6) is deformable by filling with the pressure medium and/or removing the pressure medium.

8. Preparation station (7) according to either of the preceding claims 6 or 7, wherein the cleaning covering (16) can be placed, preferably stretched, on the cleaning main body (15), preferably along an outer contour (17) of the cleaning main body (15), by means of the placement device.

9. Preparation station (7) according to any of the preceding claims 6 to 8, wherein the placement device comprises a placement drive and a connecting device for a, preferably detachable or non-detachable, connection to the cleaning covering (16), wherein the cleaning covering (16) connected to the connecting device can be moved onto the cleaning main body (15), preferably by being unrolled from a roll, by means of the placement drive until an operating position is reached.

10. Preparation station (7) according to any of the preceding claims 6 to 9, wherein the cleaning product can be applied onto and/or into the cleaning covering (16) by means of the application device (32) before, during and/or after a placement of the cleaning covering (16) on the cleaning main body (15).

11. Preparation station (7) according to any of the preceding claims 5 to 10, wherein a transfer device for transferring the cleaning unit (1) between the preparation station (7) and a medical imaging device (4) is provided, wherein the transfer device is drivable by hand and/or motor driven.

12. Preparation station (7) according to any of the preceding claims 5 to 11, wherein a displacement device (18) is provided, wherein the cleaning unit (1) detachably connected to the preparation station (7) is movable toward the medical imaging device (4) and/or away from the medical imaging device (4) and/or in at least one direction (21, 22), preferably in at least two directions, within the medical imaging device (4) by means of the displacement device (18).

13. Preparation station (7) according to any of the preceding claims 5 to 12, wherein the support structure (5) is dimensionally stable and/or the cleaning component (6) is elastically deformable.

14. Arrangement having a preparation station (7) as claimed in any of the preceding claims 5 to 13 and having a medical imaging device (4), wherein the medical imaging device (4) comprises a scanner unit (20), a patient-accommodating region (2) at least partly surrounded by the scanner unit (20) and a housing (3) that surrounds the patient-accommodating region (2), and having a cleaning unit (1) for cleaning the housing (3).

15. Arrangement according to claim 14, having a displacement device (18), preferably with a patient table (19), separate from the preparation station (7), wherein the cleaning unit (1) can be transferred from the preparation station (7) to the separate displacement device (18), preferably to the patient table (19), and/or the cleaning unit (1) can be transferred from the separate displacement device (18), preferably from the patient table (19), to the preparation station (7).

## Revendications

1. **Procédé** de préparation d'une unité de nettoyage (1) et de nettoyage d'une enveloppe (3) entourant une zone de réception de patient (2) d'un dispositif d'imagerie médicale (4)
∘ au moyen de l'unité de nettoyage (1), l'unité de nettoyage (1) présentant une structure porteuse (5) et un composant de nettoyage (6), et
∘ au moyen d'une station de préparation (7), de préférence séparée du dispositif d'imagerie médicale (4) et disposée à proximité de celui-ci, comportant au moins un dispositif de préparation et au moins un dispositif de déplacement (18), l'unité de nettoyage (1) étant reliée de manière détachable à la station de préparation (7),
∘ au moins une étape de préparation étant effectuée par ledit au moins un dispositif de préparation, par laquelle l'unité de nettoyage (1) est préparée pour un nettoyage ultérieur, et
∘ le nettoyage étant effectué par un déplacement, à l'intérieur du dispositif d'imagerie médicale (4), de l'unité de nettoyage (1) préparée dans au moins une direction (21, 22), de préférence dans au moins deux directions.

2. Procédé selon la revendication 1, dans lequel
- l'unité de nettoyage (1) est déplacée vers la station de préparation (7), et
- ensuite au moins une étape de préparation est effectuée, par laquelle l'unité de nettoyage (1) est préparée pour un nettoyage ultérieur.

3. Procédé selon la revendication 1 ou 2 comportant au moins l'une des étapes suivantes consistant à :
- relier de façon détachable l'unité de nettoyage (1) à la station de préparation (7), de préférence au moyen d'un dispositif de couplage (30),
- recharger un accumulateur de force et/ou un accumulateur d'énergie,
- déformer, de préférence gonfler, le composant de nettoyage (6) par remplissage avec un fluide sous pression ou par évacuation dudit fluide sous pression,
- placer, de préférence tendre, un support de nettoyage (16) du composant de nettoyage (6) sur un corps de base de nettoyage (15) du composant de nettoyage (6), de préférence le long d'un contour extérieur (17) du corps de base de nettoyage (15),
- appliquer et/ou introduire un agent de nettoyage sur et/ou dans un support de nettoyage (16) du composant de nettoyage (6), de préférence avant, pendant et/ou après un placement du support de nettoyage (16) du composant de nettoyage (6) sur un corps de base de nettoyage (15) du composant de nettoyage (6).

4. Procédé selon l'une des revendications précédentes comportant au moins l'une des étapes suivantes consistant à :
- détacher l'unité de nettoyage (1) reliée de manière détachable à la station de préparation (7), de préférence au moyen du dispositif de couplage (30),
- transférer l'unité de nettoyage (1) entre le dispositif d'imagerie médicale (4) et la station de préparation (7),
- relier de manière détachable l'unité de nettoyage (1) au dispositif d'imagerie médicale (4),
- détacher l'unité de nettoyage (1) du dispositif d'imagerie médicale (4).

5. Station de préparation (7) pour la préparation d'une unité de nettoyage (1), l'unité de nettoyage (1) étant prévue pour nettoyer une enveloppe (3) entourant une zone de réception de patient (2) d'un dispositif d'imagerie médicale (4), l'unité de nettoyage (1) présentant une structure porteuse (5) et un composant de nettoyage (6), la station de préparation (7) étant séparée du dispositif d'imagerie médicale (4) et disposée à proximité de celui-ci, la station de préparation (7) comportant au moins un dispositif de préparation à l'aide duquel l'unité de nettoyage (1) est préparée pour un nettoyage ultérieur.

6. Station de préparation (7) selon la revendication 5, dans laquelle ledit au moins un dispositif de préparation est
- un dispositif de couplage (30) pour relier de manière détachable l'unité de nettoyage (1) à la station de préparation (7),
- un dispositif de rechargement pour recharger un accumulateur de force et/ou un accumulateur d'énergie,
- un dispositif de déformation (12) pour déformer le composant de nettoyage (6),
- un dispositif de placement pour placer un support de nettoyage (16) du composant de nettoyage (6) sur un corps de base de nettoyage (15) du composant de nettoyage (6), ou
- un dispositif d'application (32) pour appliquer un agent de nettoyage sur et/ou dans un support de nettoyage (16) du composant de nettoyage (6).

7. Station de préparation (7) selon la revendication 6, dans laquelle le dispositif de déformation (12) comporte un entraînement de déformation (13), un fluide sous pression et une conduite de liaison (14) pour relier, de préférence de manière détachable ou non détachable, le composant de nettoyage (6) à l'entraînement de déformation (13), le fluide sous pression pouvant être introduit dans le composant de nettoyage (6) et/ou retiré du composant de nettoyage (6) au moyen de l'entraînement de déformation (13), le composant de nettoyage (6) étant déformable par remplissage avec le fluide sous pression et/ou par évacuation dudit fluide sous pression.

8. Station de préparation (7) selon l'une des revendications 6 ou 7, dans laquelle le support de nettoyage (16) peut être placé, de préférence peut être tendu, au moyen du dispositif de placement sur le corps de base de nettoyage (15), de préférence le long d'un contour extérieur (17) du corps de base de nettoyage (15).

9. Station de préparation (7) selon l'une des revendications 6 à 8, dans laquelle le dispositif de placement comporte un entraînement de placement et un dispositif de liaison pour le relier, de préférence de manière détachable ou non détachable, au support de nettoyage (16), le support de nettoyage (16) relié au dispositif de liaison pouvant être déplacé par l'entraînement de placement jusqu'à une position de service sur le corps de base de nettoyage (15), de préférence pouvant être déroulé à partir d'un rouleau.

10. Station de préparation (7) selon l'une des revendications 6 à 9, dans laquelle l'agent de nettoyage peut être appliqué au moyen du dispositif d'application (32) sur et/ou dans le support de nettoyage (16) avant, pendant et/ou après un placement du support de nettoyage (16) sur le corps de base de nettoyage (15).

11. Station de préparation (7) selon l'une des revendications 5 à 10, dans laquelle un dispositif de transfert est prévu pour transférer l'unité de nettoyage (1) entre la station de préparation (7) et un dispositif d'imagerie médicale (4), le dispositif de transfert pouvant être entraîné manuellement et/ou étant motorisé.

12. Station de préparation (7) selon l'une des revendications 5 à 11, dans laquelle un dispositif de déplacement (18) est prévu, l'unité de nettoyage (1) reliée de manière détachable à la station de préparation (7) pouvant, au moyen du dispositif de déplacement (18), être déplacée vers le dispositif d'imagerie médicale (4) et/ou écartée du dispositif d'imagerie médicale (4) et/ou déplacée à l'intérieur du dispositif d'imagerie médicale (4) dans au moins une direction (21, 22), de préférence dans au moins deux directions.

13. Station de préparation (7) selon l'une des revendications 5 à 12, dans laquelle la structure porteuse (5) est rigide et/ou le composant de nettoyage (6) est déformable de manière élastique.

14. Ensemble comprenant une station de préparation (7) selon l'une des revendications 5 à 13 et un dispositif d'imagerie médicale (4), le dispositif d'imagerie médicale (4) comportant une unité de scanner (20), une zone de réception de patient (2) entourée au moins partiellement par l'unité de scanner (20), et une enveloppe (3) entourant la zone de réception de patient (2), et comprenant une unité de nettoyage (1) pour nettoyer l'enveloppe (3).

15. Ensemble selon la revendication 14, comprenant un dispositif de déplacement (18) séparé de la station de préparation (7), de préférence avec une table patient (19), l'unité de nettoyage (1) pouvant être transférée de la station de préparation (7) vers le dispositif de déplacement (18) séparé, de préférence vers la table patient (19), et/ou du dispositif de déplacement (18) séparé, de préférence de la table patient (19), vers la station de préparation (7).
